# EUROPÄISCHE PATENTSCHRIFT

(11) **EP 3 448 434 B1**
(45) Veröffentlichungstag und Bekanntmachung des Hinweises auf die Patenterteilung: **21.10.2020**
(21) Anmeldenummer: 17722702.2
(22) Anmeldetag: 26.04.2017
(51) Int. Cl.: A61K 47/34, C09D 1/04, A23P 20/10

(54) **ESSBARE FUNKTIONSSCHICHTEN UND ÜBERZÜGE AUF HYBRIDPOLYMERBASIS FÜR PHARMAZIE UND LEBENSMITTEL**
EDIBLE FUNCTIONAL LAYERS AND COATINGS BASED ON HYBRID POLYMERS FOR PHARMACEUTICALS AND FOOD
COUCHES FONCTIONNELLES COMESTIBLES ET ENROBAGES À BASE DE POLYMÈRE HYBRIDE EMPLOYÉS DANS LE DOMAINE PHARMACEUTIQUE ET ALIMENTAIRE

(30) Priorität: 26.04.2016 DE 102016107760
(43) Veröffentlichungstag der Anmeldung: 06.03.2019
(73) Patentinhaber: Fraunhofer-Gesellschaft zur Förderung der angewandten Forschung e.V., 80686 München (DE)
(72) Erfinder: AMBERG-SCHWAB, Sabine, 97250 Erlabrunn (DE); COLLIN, Daniela, 97440 Werneck (DE); DEINHARDT, Anika, 97534 Waigolshausen (DE)
(74) Vertreter: Strehl Schübel-Hopf & Partner
(86) Internationale Anmeldenummer: PCT/EP2017/059873
(87) Internationale Veröffentlichungsnummer: WO 2017/186767

(56) Entgegenhaltungen:
- EP-A2- 0 802 218
- EP-A2- 1 112 738
- US-A1- 2007 141 104
- PINGGUI LIU ET AL: "Microstructure and thermal properties of silyl-terminated polycaprolactone?polysiloxane modified epoxy resin composites", JOURNAL OF APPLIED POLYMER SCIENCE, Bd. 109, Nr. 2, 1. Januar 2008 (2008-01-01), Seiten 1105-1113, XP55387949, US ISSN: 0021-8995, DOI: 10.1002/app.28292 in der Anmeldung erwähnt

## Beschreibung

### Gebiet der Erfindung

Die vorliegende Erfindung betrifft die Verwendung einer Zusammensetzung, die mit organischen Gruppen modifiziertes Kieselsäure(hetero)polykondensat enthält, wobei die organischen Gruppen Polymere sind, als Beschichtung von Arzneimitteln und Lebensmitteln. Außerdem betrifft die vorliegende Erfindung ein Verfahren zur Herstellung eines Beschichtungsprodukts sowie ein Beschichtungsprodukt.

### Hintergrund der Erfindung

### 1. Medikamente

Um Medikamente zu verpacken, wurden bislang z. B. PVDC (Polyvinylidenchlorid) und Aluminium-Verbundfolien eingesetzt, damit die Wirkstoffe ausreichend vor Feuchtigkeit, Sauerstoff, UV-Strahlung und mechanischer Beschädigung geschützt werden können.

Um zu vermeiden, dass für Pharmaverpackungen chlorhaltige Folien und Aluminiumverpackungen eingesetzt werden, hat man nach alternativen Materialien gesucht, die bei gleichbleibend guten Barriereeigenschaften oft auch tiefziehfähig sein müssen.

Um die sehr hohen Anforderungen an die Barriere von pharmazeutischen Verpackungen zu erfüllen, werden zumeist PVDC oder PVC als Verbundfolie genutzt. Deren Wasserdurchlässigkeit liegt in der Regel für flache Folien zwischen 0,09 g/m²·d (z.B. für 51 µm PCTFE (Polychlortrifluorethylen) auf PVC und 4 g/m²·d (z.B. für 250 µm dickes PVC). Nach Thermoformen steigt sie auf das ca. 2,5-3,5-fache.

Ergänzend zu den Barriereeigenschaften der Verpackung werden Medikamente bzw. Tabletten zudem auch selbst beschichtet. Diese Beschichtungen erfüllen neben zusätzlicher Barrierewirkung weitere Aufgaben (s.u.). Allerdings ist die Barrierewirkung des Tablettenüberzugs alleine nicht ausreichend wirksam.

Überzüge für Tabletten und Medikamente haben allgemein sehr unterschiedliche Aufgaben zu erfüllen. Sie dienen z.B.:
- dem Schutz der Wirkstoffe gegen Licht, Luftsauerstoff und Feuchtigkeit und beugen somit einer Alterung vor
- der mechanischen Stabilisierung während Herstellung, Verpackung und Versand
- dem Schutz des Wirkstoffs gegen den Einfluss von Verdauungssäften (pH-abhängige Löslichkeit)
- der gesteuerten Freisetzung im humanen Körper; Löslichkeit angepasst an die beabsichtigte Verwendung
- der Vermeidung von Nebenwirkungen des Wirkstoffs
- der Identifizierung verschiedener Arzneimittel durch farbliche Unterscheidung der Tabletten
- Verbesserung der Schluckeigenschaften
- wenn das Medikament als Suspension (aufgelöst zumeist in Wasser) eingenommen werden soll, ist eine gute Löslichkeit oder Dispergierbarkeit wichtig
- Möglichkeit, eine ästhetische/schöne Oberfläche zu bilden
- Geruchs-, Geschmacks- und Farblosigkeit sowie gesundheitliche Unbedenklichkeit
- Kompatibilität zu den Hilfs- und Wirkstoffen im Kern
- Kompatibilität mit den gängigen Filmzusätzen wie Weichmacher, Farb- und Füllstoffe
- keine Auffüllung der Gravur.

Die Auswahl des eingesetzten Polymerfilms für die Beschichtung von Pharmazeutika hängt u.a. davon ab, wo und in welcher zeitlichen Spanne der Wirkstoff freigesetzt werden soll. Ist eine schnelle Freisetzung (fast release) gewünscht, können die Kerne mit magensaftlöslichen Polymeren, die sich in einem pH-Milieu von 1 - 3,5 auflösen, überzogen werden. Wirkstoffe, die verzögert freigesetzt (sustained release) werden oder eine Empfindlichkeit gegenüber der Magensaftsäure aufweisen, werden dagegen mit Polymerschichten überzogen, die sich bei einem pH-Wert von 6,5 - 8,0 auflösen oder aufquellen, damit der Wirkstoff durch den gequollenen Film diffundieren kann.

Keines der bis heute bekannten Polymere erfüllt alle Wunschkriterien für Überzugsmaterialien gleichzeitig. Deshalb werden die Polymere häufig jeweils für die drei Hauptanwendungen des jeweiligen Medikaments ausgewählt und mit anderen Hilfsstoffen wird versucht, passende Eigenschaftskombinationen zu realisieren.

Im Folgenden werden die Stand-der-Technik-Lösungen für zunächst magensaftresistente und nicht-resistente Überzüge diskutiert, gefolgt von Beschichtungen für eine kontrollierte Freisetzung und ästhetische Überzüge.

Magensaftresistent überzogene Dragees, Tabletten, Kapseln, Pellets und Granulate spielen in der Gruppe der festen peroralen Arzneiformen seit langem eine große Rolle. Gängige Filmüberzüge basieren beispielsweise auf Methylcellulose oder anderen Cellulosederivaten mit unterschiedlichen Magensaftlöslichkeiten.

Um die Freisetzung der im Medikamentenkern eingeschlossenen Wirkstoffe über einen definierten Zeitraum kontrollieren zu können, werden ebenfalls Cellulosederivate verwendet. Diese Überzüge ermöglichen durch eine gesteuerte Degradation eine kontrollierte Wirkstofffreigabe.

Beim ästhetischen Beschichten werden nahezu ausschließlich Polymere verwendet, die gut und schnell wasserlöslich sind. Etwa 60-70% der Überzüge enthalten Hydroxypropylmethylcellulose (HPMC).

Es hat sich gezeigt, dass mit Stand-der-Technik-Tablettenüberzügen neben den bereits diskutierten Eigenschaften, wie Magensaftresistenz, kontrollierte Wirkstofffreisetzung oder Ästhetik die Wasserdampfbarriere bereits verbessert werden kann [Shen, Elizabeth. "Cover Up." Innovations in Pharm Tech 52 (2015): 44-47]. Diese reicht aber bei weitem nicht aus, um zukünftig am Verpackungsmaterial einsparen zu können, wie oben bereits diskutiert. Modifiziertes PVA hat eine sehr gute initiale Wasserdampfbarriere. Das Material besitzt trotzdem ein Wasseraufnahmevermögen, wodurch die Beschichtung aufquillt und an Barriere verliert, wenn es über längere Zeit einem feuchten Milieu ausgesetzt ist. Daraus ergibt sich jedoch, dass am hochwertigen Verpackungsmaterial mit exzellenter Barrierewirkung nicht eingespart werden darf.

Neben den zuvor beschriebenen bereits im Einsatz existierenden Beschichtungslösungen gibt es in der Patent- und Fachartikelliteratur zahlreiche Arbeiten zur Optimierung der Barriereeigenschaften durch Beschichtung der Tabletten mit neuen Material(konzepten). Diese werden im Folgenden zusammengefasst EP 1 112 738 A2 offenbart Simethicon als Bestandteil in einer Beschichtung für Arzneimittel.

EP 0 839 008 B1 beschreibt eine essbare anorganische Beschichtung auf Lebensmitteln und pharmazeutischen Produkten, sowie die Herstellung dieser. Diese anorganische Beschichtung (Bestandteile: SiO₂, SiO, CaO, ZnO, TiO₂, MnO) dient als Barriereschicht gegen Wasserdampf und Sauerstoff. Sie soll die Lagerungsbeständigkeit verbessern, Geschmack und Textur des eigentlichen Produkts aber nicht beeinflussen. Nachteile dieser im Vakuum abgeschiedenen Beschichtung sind, dass sie zum einen sehr dünn sein muss (idealerweise zwischen 0,0005 - 0,02 µm), um Rissbildung oder Abplatzen zu vermeiden und zum anderen ist der Beschichtungsprozess mittels Sputtern oder CPD sehr teuer, komplex und zeitaufwändig.

US 3 471 304 A, US 4 661 359 A und US 4 710 228 A offenbaren essbare Feuchte-Barrierebeschichtungen aus Schellack sowie Celluloseether, Monoglycerin, Metallsalzen und Fettsäuren als Überzugsmittel für Lebensmittelprodukte und als magensaftresistentes Überzugsmittel für Medikamente zur verzögerten Freisetzung oder zur Freisetzung im Darm. Diese erfüllen nicht die nötigen Barriereanforderungen für die Pharmaindustrie.

In vielen Patenten (z. B. WO 2015/031663 A1) werden als Feuchtebarriere-Zusatz Lipide, Fette, Öle und Fettsäuren angegeben. Diese haben bereits eine natürliche hydrophobe Wirkung. Die Barrierewirkung ist aber bei weitem nicht mit den ORMOCER®-Barrierelacken zu vergleichen und daher nicht alleine ausreichend. Die angegebene Wasseraufnahme ist beispielsweise max. 1% wenn bei 40°C und 75% r. F. für 30 min ausgelagert wird.

Abbaspour MR, Sharif Makhmalzadeh B, Jalali S; Jundishapur Journal of Natural Pharmaceutical Products 2010; 5(1): 6- 17 offenbart, dass Feuchtebarriereschichten eine geringe Permeabilität gegenüber Wasserdampf haben sollten, sich im Körper aber auch schnell auflösen sollten und daher gut wasserlöslich sein müssen. Bisher ist kein Material bekannt, welches beide Aspekte erfüllt, daher ist der industrielle Ansatz üblicherweise ein Kompromiss zwischen einer guten Wasserdampfbarriere und einer guten Löslichkeit in Wasser. Dies wird zumeist erreicht durch wasserlösliche Polymerfilme als Filmbildner und den Einbau von Partikeln mit hydrophoben oder Scavenger-Eigenschaften.

Dies kann z.B. Stearinsäure (durch Verseifung aus pflanzlichen und tierischen Ölen und Fetten gewonnen) sein, oder als Feuchtigkeits-Scavenger agieren, wie mikrokristalline Cellulose, welche wie ein molekularer Schwamm in Kontakt mit Wasser wirkt. Die Nachteile dieser Entwicklung ist zum einen, dass durch den Einbau von mikrokristalliner Cellulose die Beschichtung opak bzw. weiß wird. Außerdem ist die Schichtdicke mit 100 - 200 µm aus wirtschaftlicher Sicht sehr uninteressant. Die Wasserdampfbarriere verbesserte sich nur geringfügig.

Unalan IU, Cerri G, Marcuzzo E, Cozzolinoa CA, Farris S; RSC Adv., 2014, 4, 29393 offenbart, dass durch die Einbettung von Nanopartikeln in die Beschichtung sich der Diffusionsweg erhöht und dadurch sich die Barrierewirkung verbessert. Für pharmazeutische Produkte und Lebensmittel ist der Einsatz von Nanopartikeln aber eher kritisch zu betrachten und sollte wenn möglich ausgeschlossen sein, da eine Diffusion dieser Partikel in Zellen nicht vollständig ausgeschlossen ist. In der Literatur wird dieses Konzept für die Verpackung an sich beschrieben.

WO 12/116814 beschreibt die Abscheidung von anorganischen Schichten, wie Metalloxiden, auf pharmazeutische Produkten mittels ALD (atomic layer deposition). Dieser Prozess ist jedoch nicht nur sehr teuer und aufwändig (Vakuumprozess), sondern homogene Schichten auf 3D-Objekten sind nur schwer zu generieren, insbesondere wenn es in die Massenproduktion geht. Rein anorganische Beschichtungen sind zusätzlich auch sehr spröde und neigen zur Rissbildung und zum Abplatzen.

US 2015/0250731 A1 beschreibt eine Methode zur Tablettenbeschichtung und für eine pharmazeutische Formulierung. Die Beschichtung wird mittels ALD (Atomic Layer Deposition) auf die pharmazeutischen Substrate appliziert. Ein ähnliches Verfahren - MLD (molecular layer deposition) eignet sich nach Angaben im Patent zur Abscheidung hybrider organischanorganischer Schichten, welche vorab durch einfaches Mischen kombiniert werden. Als Prekursoren werden anorganische Oxide verwendet. Auch Biomaterialien wie Hydroxyapatit, Polymere, Zucker, Nanolaminate und dergleichen können darüber abgeschieden werden. Hier werden die gewünschten Prekursoren einfach nur im gewünschten Verhältnis gemischt.

Zusammenfassend zeigt die obige Betrachtung des Standes der Technik, dass die bekannten Tabletten-Beschichtungen bei weitem nicht das hohe Anforderungsprofil in Bezug auf die Barrierewirkung erfüllen. Es besteht daher ein hoher Bedarf an verbesserten Barrierebeschichtungen.

### 2. Lebensmittel

Bei Lebensmitteln gibt es im Vergleich zu Medikamenten verschiedene Beschichtungskonzepte, da die Anforderungen oft sehr vielfältig und spezifisch sind. Generell ist aber auch hier der Bedarf und Wunsch durch geeignete Barriereschichten die Haltbarkeit der Produkte zu verbessern. Beispielsweise kommt es bei Sauerstoffeintritt durch die Barriereschicht zu einer Oxidation von Fetten ("Ranzigkeit"), zu Vitaminabbau und zu Aromazerstörung. Bei Wasserdampfeintritt kann es zu Verlust der Knusprigkeit, zu einem Verklumpen hygroskopischer Produkte und zu einem Wachstum von Mikroorganismen kommen.

Im Gegensatz zu den Tablettenbeschichtungen ist bei Lebensmitteln aber insbesondere das optische Erscheinungsbild wichtig. Im Idealfall soll dem Kunden nicht bewusst sein, dass das Produkt auf dem chemischen Weg optimiert wurde. Daher sind auch in diesem Fall funktionelle Beschichtungen mit hoher Barrierewirkung und weiteren Funktionalisierungs- und Anpassungsmöglichkeiten von Vorteil.

Für Lebensmittelanwendungen können die Beschichtungen durch verschiedene Methoden auf dem Lebensmittel aufgebracht werden, wie z.B. durch Tauchen, Sprühen, Pinselauftrag und Schwenken, gefolgt von einem Trocknungsprozess.

Essbare Beschichtungen werden bisher in vier Kategorien unterteilt: Polysaccharide, Lipide, Proteine und Komposite.

Komposite sind essbare Filme und Beschichtungen und bestehen oft aus einer Mischung aus Polysacchariden, Proteinen und/oder Lipiden.

WO 2013/087757 A1 offenbart eine essbare Beschichtung, wobei die erste Schicht aus einem essbaren Öl besteht und einer zweiten Schicht mit hydrophoben essbaren Partikeln (Durchmesser 20nm - 500 µm). Die Partikel bestehen entweder aus einem anorganischen Kernmaterial und einer hydrophoben Hülle (Fette, Öle, etc), oder gänzlich aus einem hydrophoben Material wie Wachs. Anwendungen sind für Kartoffelchips, Kekse, Cornflakes, Früchte, Eiscremewaffeln etc. Dadurch saugen sich beispielsweise Cornflakes nicht so schnell mit Milch voll und haben immer noch einen sehr guten Crunch.

US 5 741 505 A offenbart eine anorganische Beschichtung auf Lebensmitteln und pharmazeutischen Produkten als Feuchte-/ Gasbarriere. Diese anorganischen Materialien für die Beschichtung beinhalten SiO₂, SiO, MgO, CaO, TiO₂, ZnO und MnO. Um Rissbildung zu verhindern, sollte die Beschichtung dünner als 0,05 µm sein. Diese dünnen Schichten verbessern die Haptikproblematik, welche durch die bisweilen dicken organischen Beschichtungen aufgetreten sind. Als mögliche Anwendung werden Cornflakes genannt, welche sich in der Milch durch die Beschichtung nicht so schnell vollsaugen.

WO 2003/007736 A1 offenbart eine kontinuierliche Fettschicht mit 1-15% wasserunlöslichen und fettunlöslichen Partikel (Durchmesser 0,05 - 100 µm). Diese Partikel sind anorganische Bestandteile SiO₂, Silikate, Talk, Ton-Materialien und Phosphate. Zusätzlich können organische Materialien wie mikrokristalline Cellulose und unlösliche Cellulose-Derivate verwendet werden.

US 2010 062 116 offenbart eine Beschichtung von mikrowellengeeigneten Speisen für knuspriges Erscheinungsbild. Einbau eines Suszeptors in die Beschichtung, welcher die Mikrowellenstrahlung in Hitze umwandelt. Hierdurch wird der Mechanismus der Mikrowellenerwärmung umgewandelt zu einem der mit einem konventionellen Ofenheizprozess vergleichbar ist. Die Zusammensetzung der Beschichtung enthält mindestens ein Prolamin und mindestens ein Hydrokolloid oder Geliermittel.

Magensaftresistent überzogene Dragees, Tabletten, Kapseln, Pellets und Granulate spielen in der Gruppe der festen peroralen Arzneiformen seit langem eine große Rolle. Magensaftresistenz wird heute erzielt durch Umhüllung der Arzneiform mit sauren Polymeren, die im Magen protoniert vorliegen und in dieser Form unlöslich sind. Erst im neutralen bis schwach basischen Milieu des Dünn- bzw. Dickdarms dissoziieren die sauren Gruppen, wodurch das Polymer in seine ionische und daher lösliche Form überführt wird. Magensaftresistente Überzüge dienen dazu, die Magenschleimhaut vor aggressiven Arzneistoffen zu schützen, die vorzeitige Inaktivierung säurelabiler Arzneistoffe zu verhindern, Arzneistoffe gezielt im Dünn- oder Dickdarm freizusetzen oder unangenehme Geschmacks-und Geruchseigenschaften von Arzneistoffen abzudecken.

Überzüge auf Lebensmitteln können eine Abgabe von Geruchsstoffen daraus verhindern und darüber hinaus die Lebensmittel vor Feuchtigkeit und unerwünschte Oxidationsreaktionen bewirkendem Sauerstoff schützen.

### Durch die Erfindung zu lösende Aufgaben

Durch den vorstehend beschriebenen Stand der Technik ergibt sich die Aufgabe, hervorragende Barrierewerte, die zum Schutz von Medikamenten oder Lebensmitteln notwendig sind, zu erzielen und gleichzeitig chlorhaltige bzw. aluminiumhaltige Verpackung und Beschichtungen überflüssig zu machen sowie auf andere teure oder bedenkliche Verfahren verzichten zu können.

Eine weitere Aufgabe ist die Bereitstellung von Feuchtebarriereschichten, die eine gute Ausgewogenheit zwischen einer geringen Permeabilität gegenüber Wasserdampf und schnellen Auflösbarkeit im Körper und daher guter Wasserlöslichkeit aufweisen.

Eine weitere Aufgabe der vorliegenden Erfindung liegt darin, Arzneimittel und Lebensmittel so herzurichten, dass die für sie einsetzbaren Verpackungsmaterialien nicht notwendigerweise besonders gute Barriereeigenschaften besitzen müssen, die Arzneimittel bzw. Lebensmittel aber dennoch ausreichend gegen Feuchtigkeit und Gase wie oxidierenden Sauerstoff geschützt sind. Denn Verpackungsmaterialien werden häufig einem Tiefziehprozess unterzogen. Tiefgezogene Verpackungsmaterialien besitzen jedoch weniger gute Barriereeigenschaften als nicht tiefgezogene.

Eine weitere Aufgabe der vorliegenden Erfindung ist es, ein Material für die Lebensmittel- und Pharmaindustrie bereitzustellen, das eine funktionelle Beschichtung darstellt. Das Material bzw. der Schichtverbund kann biologisch abbaubar sein und erst bei Kontakt mit Feuchtigkeit einen antimikrobiellen Effekt entwickeln, wobei diese Beschichtung vorzugsweise gute Barriereeigenschaften gegenüber dem Durchtritt von Sauerstoff und Wasserdampf besitzt, um den Inhalt vor z.B. oxidativen Prozessen zu schützen.

### Lösung der Aufgabe

Die genannte Aufgabe wird dadurch gelöst, dass bereits in einer Beschichtung des Medikaments bzw. des Lebensmittels die vorteilhaften und gewünschten Eigenschaften realisiert werden.

Erfindungsgemäß wird die Verwendung einer Zusammensetzung, die mit organischen Gruppen modifiziertes Kieselsäure(hetero)polykondensat enthält, wobei die organischen Gruppen Polymere sind, als Beschichtung eines unter einem Arzneimittel und Lebensmittel ausgewählten Substrats oder als Komponente einer solchen Beschichtung bereitgestellt, wobei die organischen Gruppen des Kieselsäure(hetero)polykondensats zumindest teilweise biologisch abbaubar sind.

Die vorliegende Erfindung stellt außerdem ein Verfahren zur Herstellung eines Beschichtungsprodukts bereit, welches folgende Stufen umfasst:
(i) das Auftragen einer Zusammensetzung, die optional in einem Verdünnungs- und/oder Lösungsmittel vorliegt und mit organischen Gruppen modifiziertes Kieselsäure(hetero)polykondensat enthält, wobei die organischen Gruppen Polymere sind, auf ein unter einem Arzneimittel und Lebensmittel ausgewähltes Substrat;
(ii) das Trocknen und/oder das Aushärten der Zusammensetzung.

Die bei der Herstellung des Beschichtungsprodukts verwendete Zusammensetzung ist vorzugsweise die Zusammensetzung, die im Zusammenhang mit der erfindungsgemäßen Verwendung eingesetzt wird.

Die vorliegende Erfindung stellt außerdem ein durch das vorstehend genannte Verfahren erhaltenes Beschichtungsprodukt bereit sowie ein Beschichtungsprodukt, das ein unter einem Arzneimittel und Lebensmittel ausgewähltes Substrat und eine auf das Substrat aufgebrachte Beschichtung einer Zusammensetzung aufweist, die mit organischen Gruppen modifiziertes Kieselsäure(hetero)polykondensat enthält, wobei die organischen Gruppen Polymere sind.

Der hier verwendete Ausdruck "Substrat" ist nicht besonders eingeschränkt und bedeutet jegliches Arzneimittel oder Lebensmittel, das beschichtet werden kann. Voraussetzung für die Beschichtbarkeit ist ein fester Zustand oder ein Zustand, bei dem zumindest eine definierte und stabile Oberfläche vorhanden ist.

Der hier verwendete Ausdruck "Arzneimittel" oder "Medikament" bezeichnet jedes Arzneimittel oder Medikament für Menschen und Tiere, welches die genannten Voraussetzungen für ein Substrat erfüllt. Dabei umfasst ein Arzneimittel sowohl verschreibungspflichtige Medikamente als auch frei verkäufliche Mittel, wie Hustenpastillen. Die Aufnahme in den menschlichen oder tierischen Körper umfasst nicht nur die orale Aufnahme und das nachfolgende Schlucken, sondern auch die Aufnahme über die Nase oder sonstige Körperöffnungen sowie die Aufnahme in den Mund und das anschließende Lutschen oder Auflösenlassen im Mund oder Kauen ohne Schlucken. Die Aufnahme über eine sonstige Körperöffnung ist beispielsweise die rektale Aufnahme von Zäpfchen. Typische Beispiele von Arzneimitteln sind feste peroralen Arzneiformen, wie magensaftresistent überzogene Dragees, Tabletten, Kapseln, Pellets und Granulate.

Der hier verwendete Ausdruck "Lebensmittel" bezeichnet jedes Lebens- oder Nahrungsmittel für Menschen oder Tiere oder sonstiges Mittel zur Aufnahme in den menschlichen oder tierischen Körper, welches die genannten Voraussetzungen für ein Substrat erfüllt. Ein sonstiges Mittel ist zum Beispiel ein Nahrungsergänzungsmittel oder andere Supplemente, wie Vitamine oder Mineralien. Die Aufnahme in den menschlichen oder tierischen Körper umfasst nicht nur die orale Aufnahme und das nachfolgende Schlucken, sondern auch die Aufnahme in den Mund und das anschließende Lutschen oder Auflösenlassen im Mund oder Kauen ohne Schlucken. Die hier verwendeten Ausdrücke "Beschichtung" oder "Überzug" bzw. "beschichten" oder "überziehen" bezeichnen die Auftragung einer Substanz auf die Oberfläche ein Substrats, wobei die Oberfläche des Substrats ganz oder teilweise bedeckt werden kann. Die Dicke der Beschichtung oder des Überzugs ist nicht eingeschränkt und variiert in Abhängigkeit von der Größe des Substrats, der Beschaffenheit der Oberfläche des Substrats und der Verwendung der Beschichtung. Ein typischer Bereich der Dicke ist 1 µm bis 1 mm.

Die erfindungsgemäßen Beschichtungsmaterialien sind wie erwähnt biologisch abbaubar; sie verhalten sich im Körper unbedenklich und werden problemlos verstoffwechselt. Mit den erfindungsgemäßen Materialien wird es daher erstmals möglich, auch Schichten im Bereich der Tablettenüberzüge bzw. Lebensmittelüberzüge zur Verfügung zu stellen.

Gleichzeitig können gewünschte Eigenschaften der Schichten, z. B. Barriereeigenschaften, eingestellt werden. Dies gelingt u.a. durch eine Kombination aus einer oder mehreren Schichten aus dem erfindungsgemäßen Material mit einer oder mehreren sehr dünnen, rein anorganischen Schichten, die z.B. aufgesputtert werden.

Auch die Löslichkeit der Schichten im Magen bzw. im Dünndarm lässt sich einstellen. Die Si-O-Si-Bindungen der erfindungsgemäßen Kondensate sind in der Regel erst einmal säurestabil, aber basenlabil, so dass das damit beschichtete Produkt dann, wenn die Beschichtung vollständig ist, den Magen unverdaut passiert, aber im Dünndarm abgebaut wird. Dies ist für eine Reihe von Tablettenapplikationen wichtig. Allerdings lassen sich derartige Kondensate auch dahingehend modifizieren, dass sie säurelabil sind. Dies gelingt durch die Modifikation mit geeigneten, über Kohlenstoff an Silicium gebundenen Gruppen, wie es dem Fachmann bekannt ist. Auch durch eine Verringerung der Vernetzungsgrade, speziell des anorganischen Vernetzungsgrades (also der Si-O-Si- bzw. Si-O-Metall-Bindungen) kann die Säurelabilität eingestellt werden.

Die erfindungsgemäßen Beschichtungsmaterialien lassen sich als Lacke applizieren. Sie sind insbesondere günstig, da sich mit ihnen Barriereeigenschaften gegenüber Wasserdampf und Sauerstoff erzielen lassen.

Für die biologische Abbaubarkeit der Beschichtung ist es notwendig, dass ein möglichst großer Anteil der organischen Gruppen, die in das anorganische Netzwerk des Hybridmaterials eingebunden sind, biologisch abbaubar ist. Nach Aufnahme des Produkts in den Körper kann die Beschichtung durch Stoffwechselvorgänge in natürliche Stoffwechselprodukte, wie CO₂ oder H₂O, umgesetzt werden, Auch können Abbauprodukte in den menschlichen Metabolismus eingeschleust werden. Zurück bleiben im Wesentlichen nur die anorganischen Bestandteile des vorherigen Hybridmaterials (z. B. SiO₂), welche in Form von natürlichen Mineralien bereits in der Natur vorkommen und vom Organismus ausgeschieden werden.

Mit Hilfe der erfindungsgemäß einsetzbaren Beschichtung lassen sich hervorragende Barrierewerte für Wasserdampf und Sauerstoff erzielen, die zum Schutz der pharmazeutischen Produkte, insbesondere Tabletten, Medikamentenkerne u. dgl., bzw. Lebensmittel notwendig sind. Der Schutz von pharmazeutischen Produkten und Lebensmitteln lässt sich damit teilweise oder vollständig durch eine solche Beschichtung realisieren, was eine oft chlorhaltige bzw. aluminiumhaltige Verpackung überflüssig macht bzw. einen Verzicht auf andere teure oder bedenkliche Verfahren ermöglicht.

Die erfindungsgemäße Beschichtung wird erzeugt, indem ein entsprechender Beschichtungslack auf das gewünschte Pharmazeutikum, insbesondere eine Tablette, oder das gewünschte Lebensmittel aufgetragen wird. Enthält er Lösungsmittel, kann dieses bei Bedarf entfernt werden. Alternativ oder kumulativ ist eine thermische Nachbehandlung oder eine Bestrahlung mit Licht möglich.

Die anorganische Komponente des erfindungsgemäßen Beschichtungslacks besteht aus Silicium-Kationen, gegebenenfalls in Kombination mit weiteren Kationen wie Aluminium, Zirkonium, Titan oder Bor sowie Kombinationen davon, die über Sauerstoffbrücken miteinander verknüpft sind und dabei ein Netzwerk bilden. Damit handelt es sich um ein organisch modifiziertes Kieselsäure- oder, im Falle der Anwesenheit weiterer Metallionen, um ein organisch modifiziertes Kieselsäurehetero-Polykondensat. Ein Beispiel für ein solches Heteropolykondensat ist ein Kondensat, das Silicium- und Aluminium- sowie optional weitere Kationen enthält. Nachstehend wird als gemeinschaftlicher Begriff für die reinen Kieselsäurepolykondensate und die Heteroatome enthaltenden Polykondensate der Begriff Kieselsäure(hetero)polykondensat verwendet.

Das organisch modifizierte Kieselsäure(hetero)polykondensat wird in der Regel durch hydrolytische Kondensation von Silanen, ggf. in Kombination mit co-kondensierbaren Verbindungen anderer Metallionen, erzeugt. Diese Silane können neben hydrolysierbaren Gruppen wie Alkoxiden oder Hydroxid-Gruppen über Kohlenstoff gebundene organische Gruppen tragen. Diese Gruppen bleiben bei der hydrolytischen Kondensation an die jeweiligen Silicium-Atome gebunden; mit ihnen wird das Polykondensat in geeigneter Weise modifiziert. Aus diesem Grunde liegen die biologisch abbaubaren organischen Gruppen bevorzugt zumindest teilweise über Si-C-Bindungen in das Kondensat eingebunden vor. Das schließt jedoch keinesfalls die Möglichkeit aus, Polykondensate mit über Sauerstoff gebundenen biologisch abbaubaren, organischen Gruppen einzusetzen. Solche Polykondensate kennt der Fachmann.

In das Kieselsäure(hetero)polykondensat der vorliegenden Erfindung sind wie erwähnt organische, biologisch abbaubare Gruppen integriert. Bezüglich dieser Gruppen kann der Fachmann auf Materialien zurückgreifen, die aus dem Stand der Technik bekannt sind.

Im Folgenden werden verschiedenste Arten von Substanzen, darunter Oligo- und Polymere, genannt, die in der vorliegenden Erfindung eingesetzt werden können. Die Ansprüche offenbaren Polymere als organische Gruppen.

Aus dem Stand der Technik sind viele Polymere bekannt, die gut und schnell wasserlöslich sind. Häufig handelt es sich um Saccharid-Derivate. Beispiele sind Hydroxypropylmethylcellulosen (HPMC), Methylcellulosen (MC), Natriumcarboxymethylcellulose (NaCMC), Hydroxyethylcellulose (HEC), Hydroxypropylcellulose (HPC), Polyvinylpyrrolidon (PVP) und Polyvinylalkohol (PVA). HPMC bildet gut wasserlösliche Filme, die etwas zur Sprödigkeit neigen. MC ist ein wasserlösliches Polymer. Die hohe Viskosität der Lösung erschwert jedoch die Verarbeitung. Eine Tendenz zur Retardierung wurde auch beobachtet. NaCMC ist ein weit verbreitetes und sehr gut wasserlösliches Polymer mit stark verdickender Wirkung. Ein deutlicher Nachteil sind die teilweise unzureichenden mechanischen Eigenschaften der Filme aus NaCMC. HEC wird wegen der starken Klebrigkeit selten als Filmüberzug verwendet, sondern häufiger als Haftvermittler zugesetzt. HPC ist ein extrem klebriges Polymer, das mechanisch stabile Filme bildet. HPC wird allein kaum verwendet, als Co-Polymer jedoch deutlich besser zu verarbeiten. PVP allein bildet eine klebrige Lösung und einen spröden Film, erhöht aber den Glanz und die Farbhomogenität zusammen mit HPMC. PVA als Polymer allein ist stark klebrig. Als Co-Polymer (Kollicoat/BASF) verbessert es die mechanischen Eigenschaften.

Polysaccharide, die als die modifizierenden organischen Gruppen in der vorliegenden Erfindung eingesetzt werden können und bereits im Stand der Technik für essbare Beschichtungen oder Überzüge genutzt werden, beinhalten Cellulose, Stärke-Derivate, Pectin-Derivate, Algenextrakte, Gummiarabikum und Chitosan. Polysaccharide sind üblicherweise sehr hydrophil und haben eine schlechte Wasserdampf- und Gasbarriere. Sie werden vorwiegend als Opferschichten für Lebensmittelprodukte eingesetzt.

Cellulose und Derivate davon bieten gute Filmbildungseigenschaften, welche geruchslos, geschmacksfrei, flexibel und transparent sind. Sie haben eine moderate Festigkeit und sind beständig gegen Öle und Fette, haben jedoch eine schlechte Wasserdampfbarriere.

Chitosan kann teil-durchlässige Beschichtungen ausbilden, sodass die innere Atmosphäre modifiziert werden kann. Hierdurch wird der Reife- und Abbauprozess in Obst und Gemüse verzögert. Überzüge aus Chitosan sind transparent, robust, flexibel und haben gute Sauerstoffbarriere. CO₂ Permeabilität könnte durch Methylierung der Polymere verbessert werden. Sie sind biodegradierbar, biokompatibel und chemisch inert und darüber hinaus auch relativ günstig.

Stärke wird bei industriell produzierten Lebensmitteln verwendet, da es biodegradierbar und essbar ist, in großen Mengen anfällt, wenig kostet, nicht allergen wirkt und einfach zu verarbeiten ist. Beschichtungen mit hohem Amylose-Anteil haben eine gute SauerstoffBarriere, sind flexibel, resistent gegen Öl. Sie sind geruchslos, geschmacksfrei, transparent, biologisch absorbierbar, semipermeabel für CO₂.

Carrageenan beinhaltet einen Geliermechanismus, welcher bei einem moderaten Trocknungsprozess, ein 3-dimensionales Netzwerk aus Polysaccharid-Doppelhelixen ausbildet. Dieser feste Film wird für Desserts, Eiscreme, Milkshakes, Salat-Dressings, Kondensmilch und Saucen eingesetzt, um die Viskosität zu erhöhen. Es wird auch in Bier, Zahnpasta, Sojamilch u.v.a. Produkten eingesetzt.

Auch andere Materialien können geeignet sein. Acetylierte Glyzerinmonostearate können auf 800% ihrer ursprünglichen Länge gedehnt werden. Die Feuchtebarriere ist deutlich schlechter als bei den Polysacchariden. Einsatz für Geflügel und Fleischwaren, um den Flüssigkeitsverlust während der Lagerung zu minimieren.

Die vorgenannten Materialien können genutzt werden, um als solche an geeignete Silane oder bereits hydrolytisch kondensierte Kieselsäurehomo- und/oder -heteropolykondensate angebunden zu werden und damit biologisch abbaubare Gruppen in das Kondensat einzubinden. Alternativ können Bestandteile daraus oder Abbauprodukte davon wie einzelne Zuckerkomponenten hierfür verwendet werden. Um angebunden werden zu können, benötigen die Materialien oder Gruppen vorzugsweise mindestens eine freie OH- oder CarbonsäureGruppe, die z.B. über eine an Silicium gebundene, Isocyanat oder Epoxy enthaltende Gruppe eingebunden werden kann, wie unten näher erläutert.

Es können auch Komposite verwendet werden. Essbare Filme und Beschichtungen bestehen aus einer Mischung aus Polysacchariden, Proteinen und/oder Lipiden. Das Ziel ist es, solche Kompositfilme zu produzieren, die eine verbesserte Barriere oder mechanische Eigenschaften in Abhängigkeit von der Anwendung haben.

Bevorzugt handelt es sich dabei um eine oder mehrere Arten von organischen Monomeren, Oligomeren oder Polymeren, wie Caprolacton/Caprolactam-Polymere, z.B. Polycaprolacton, Polycaprolacton-triol oder Derivate von Polycaprolacton-triol, Polymilchsäure, biobasierte Wachse wie z.B. Cutine, Chitosan oder auch Hemicellulosen oder Cellulosen, darunter Cellulosederivate und/oder Cellulosebausteine. Unter "Cellulosebausteinen" sollen dabei alle Monomere, Oligomere und Polymere zu verstehen sein, die über mindestens zwei über eine β-1,4-glykosidische Bindung aufweisende β-D-Glucose-Einheiten und über mindestens eine OH-Gruppe verfügen. Günstig sind Gruppen, deren Kohlenwasserstoffketten durch eine oder mehrere Ester- und/oder Amid- und/oder Ether- und/oder Urethangruppen derart unterbrochen sind, dass nur wenige Kohlenstoffatome unmittelbar aufeinander folgen.

Besonders bevorzugt kommen eine oder mehrere Arten von organischen Polymeren, und zwar sowohl biobasierte biologisch abbaubare Naturstoffe und Naturstoff-Derivate wie Chitosan, Cellulose, Cellulosederivate und/oder Cellulosebausteine, als auch erdölbasierte biologisch abbaubare Edukte, z. B. Polycaprolacton-triol (PCL-T)zum Einsatz.

Ein Beispiel für einen biologisch abbaubaren Kunststoff ist petrobasiertes Polycaprolacton (PCL). Der Polyester wird aus dem zyklischen ε-Caprolacton durch eine ringöffnende Polymerisationsreaktion dargestellt. Der Thermoplast besitzt eine niedrige Glasübergangstemperatur von -60°C, was ihm eine wachsartige Konsistenz verleiht. Die gute Wasser-, Öl- und Lösungsmittelbeständigkeit macht PCL für Anwendungen aller Art, wie z. B. für Verpackungen oder als Trägermaterial für Zellen bei der Gewebezüchtung interessant. PCL kann durch das Enzym Lipase biodegradiert werden. Die optimale Abbautemperatur von PCL liegt bei 50°C (RUDNIK, E.: Compostable Polymer Materials. Amsterdam: Elsevier, 2008). Die Polymerketten des PCL enthalten maximal 5 Kohlenstoffatome, die sich jeweils zwischen einer Carboxygruppe und einer Ethergruppe (bzw. einer endständigen OH-Gruppe) befinden und fallen damit in die obigen Definition von Gruppen mit Kohlenstoffketten, die durch eine oder mehrere Ester- und/oder Amid- und/oder Ether- und/oder Urethangruppen unterbrochen sind. Bei der Hydrolyse von PCL wird das Molekül so gespalten, dass 6-Hydroxycapronsäure entsteht, welche im Zitronensäurezyklus vollständig metabolisiert wird.

Vorzugsweise ist mindestens ein Teil der organischen, biologisch abbaubaren Gruppen des erfindungsgemäß einsetzbaren Beschichtungsmaterials jeweils über ein Kohlenstoffatom an ein Siliciumatom gebunden. Da die zugrundeliegenden organischen Moleküle in der Regel freie Hydroxy- und/oder Carbonsäuregruppen aufweisen, gelingt die Anbindung in einfacher Weise z.B. durch eine Reaktion mit Silanen, die eine über Kohlenstoffatom an Silicium gebundene Gruppe tragen, welche mit einer Isocyanat-Gruppe substituiert ist. Isocyanatgruppen können sowohl mit Hydroxygruppen (unter Bildung einer Urethangruppe) als auch mit Carbonsäuregruppen (unter Bildung einer Amidgruppe und freiem CO₂) reagieren. Beispiele für geeignete Isocyanat-Silane sind Alkoxysilane der Formel RₐSiX₄₋ₐ, worin X eine hydrolyseempfindliche Gruppe oder eine Hydroxygruppe, insbesondere eine Alkoxygruppe ist, besonders bevorzugt Methoxy oder Ethoxy. R stellt eine Isocyanatoalkylgruppe dar, und a ist 1 oder 2, wobei 1 bevorzugt ist. Bei der Isocyanatoalkylgruppe kann es sich um die Isocyanatopropylgruppe handeln. Beispielsweise ist das gängige Isocyanatopropyltriethoxysilan ein geeignetes Silan für die Zwecke der Erfindung. Anstelle der Isocyanatogruppe kennt der Fachmann jedoch selbstverständlich weitere Gruppen, die, meist durch Kondensationsreaktionen, mit Carbonsäure- oder Hydroxygruppen reagieren, wie Amino- oder ggf. aktivierte Carbonsäuregruppen. Silane, die solche Gruppen über Kohlenstoff am Silicium gebunden enthalten, sind in großer Zahl bekannt und eignen sich gleichermaßen zum Anbinden der genannten organischen Moleküle.

In einer bevorzugten Ausführungsform werden über die Kupplungsreaktion mit der Isocyanatgruppe Silane erzeugt, die Caprolacton-Derivate aufweisen.

In einer ebenfalls bevorzugten Ausführungsform, die mit allen anderen Ausführungsformen der Erfindung kombinierbar ist, wird als organische, biologisch abbaubare Gruppe eine solche gewählt, in der mindestens zwei kurze Kohlenstoffketten wie oben definiert in verzweigter Stellung zueinander vorliegen. Für diese Ausführungsform hat sich insbesondere Polycaprolacton-triol (PCL-T) als geeignet erwiesen. Es handelt sich dabei um ein PCL-Derivat, das auf drei PCL-Ketten basiert, die über eine 1,1,1-Trimethylolpropan-Einheit verknüpft vorliegen: 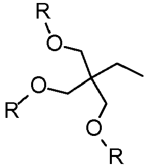 R =

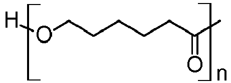

Die bei Raumtemperatur hochviskose Flüssigkeit (molekulare Masse -300 g/mol) ist biologisch abbaubar. Alle vorhandenen Hydroxygruppen des PCL-T sind bezüglich des verwendeten Reaktionspartners, dem Isocyanatsilan, gleich reaktiv. Die Anzahl der Silane, die an ein Molekül PLC-T gebunden werden kann, wird daher über die Stöchiometrie gesteuert. Erfindungsgemäß hat sich die die Funktionalisierung von einer bzw. zwei Hydroxygruppen als günstig erwiesen. Es entsteht ein Mono- oder Disilan, dessen Silanatome je nach Wahl des Index a in der Verbindung RₐSiX₄₋ₐ zwei oder drei hydrolyseempfindliche Gruppen tragen. Im nachfolgenden Formelschema wurde ein Triethoxysilan eingesetzt, also ein Silan der Formel RₐSiX₄₋ₐ, worin R Ethoxy bedeutet und a 1 ist. Es sollte aber klar sein, dass R und a auch andere Bedeutungen haben können wie oben erläutert.

Jede der Hydroxygruppen des PLC-T kann über eine Si-C-Bindung mit einem Silylrest verknüpft werden, beispielsweise über die oben erwähnte Isocyanat-Kupplung. Das Reaktionsschema zur Herstellung des mit einer Silylgruppe funktionalisierten PLC-T kann wie folgt dargestellt werden: 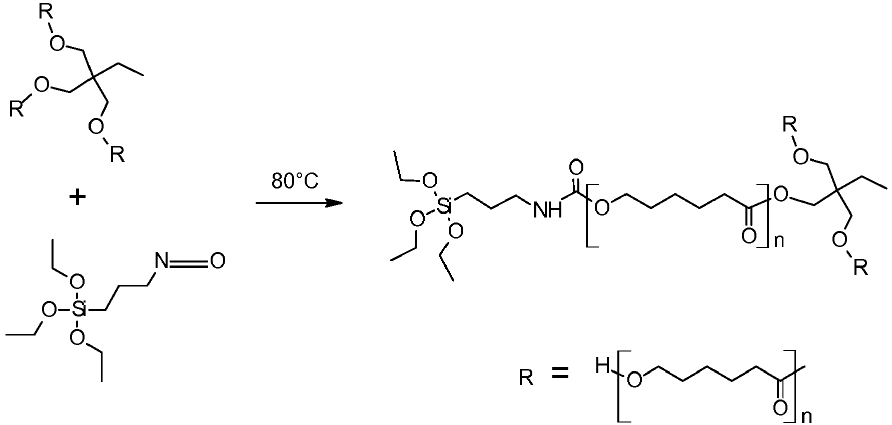 Das mit einer Silylgruppe funktionalisierte PLC-T wird nachstehend als PCL-T-1/3-triethoxysilan, das mit zwei Silylgruppen funktionalisierte PLC-T wird nachstehend als (PCL-T-2/3-triethoxysilan bezeichnet.

Alternativ lässt sich anstelle von PLC-T z.B. Cellulose in das anorganische Netzwerk einbauen. In diesen Fällen wurde Cellulose mit Epichlorhydrin funktionalisiert, wobei epoxy- bzw. diolfunktionalisierte Cellulose entsteht. Durch eine Vernetzungsreaktion mit einem epoxygruppenhaltigen Kieselsäure(hetero)polykondensat kann die Cellulose in das Kieselsäure(hetero)polykondensat eingebunden werden.

Die beiden Reaktionsvarianten zeigen zwei der vielen Möglichkeiten des Einbaus der biologisch abbaubaren Gruppen: Im ersteren Fall erfolgt eine Anbindung dieser Gruppen über eine Si-C-Bindung, im letzteren Fall über eine Anbindung an das organische Netzwerk.

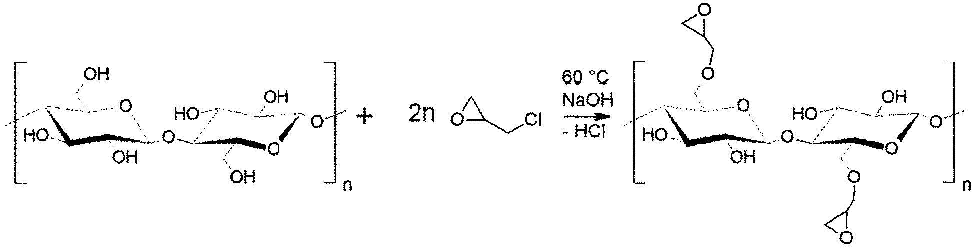

Um die Relation des anorganischen Netzwerks zum organischen Anteil zu steuern, kann die hydrolytische Kondensation der wie oben beschrieben mit organischen, biologisch abbaubaren Substituenten modifizierten Silane in Gegenwart von weiteren, hydrolytisch kondensierbaren Verbindungen der Formel M^{b}(X)_{b} durchgeführt werden, worin M ausgewählt ist unter einem Metall, das wie oben beschrieben über Sauerstoffatome in das anorganische Netzwerk des Kieselsäure(hetero)polykondensats eingebaut werden kann, also z.B. unter Al, Si, Zr, Ti, B, während b die formale Oxidationsstufe dieses Metalls darstellt und X wie für die Silane beschrieben eine hydrolyseempfindliche Gruppe oder eine Hydroxygruppe bedeutet. Beispielsweise kann als Verbindung der Formel M^{b}(X)_{b} ein Tetraalkylorthosilikat der Formel Si(OR)₄ mit R = Methyl oder Ethyl und/oder ein Aluminiumalkoholat eingesetzt werden. Letzteres kann komplexiert vorliegen, um die hydrolytische Kondensationsreaktion dieser Komponente bei der Sol-Gel-Bildung zu verlangsamen, was zur Bildung ausgedehnterer Netzwerkstrukturen führt.

Weiterhin ist es bevorzugt, dass der erfindungsgemäß einsetzbare Beschichtungslack gute Beschichtungseigenschaften besitzt. Hierfür ist es günstig, wenn auch nicht zwingend, eine oder mehrere Komponenten zuzusetzen, die die Beschichtungs- (Filmbildungs-) und/oder Barriereeigenschaften positiv beeinflussen. Das können beispielsweise organische Komponenten oder Komponenten mit einem organischen Anteil sein, die sich organisch polymerisieren lassen und damit ein zusätzliches organisches Netzwerk zur Erhöhung der Barrierewirkung der Beschichtung ausbilden können, und/oder polare Gruppen aufweisen, um die Haftung am Substrat zu verbessern. Als organisch polymerisierbare Gruppen eignen sich Gruppen mit reaktiven Ringen wie Epoxygruppen oder mit nicht-aromatischen C=C-Doppelbindungen wie (Meth-)Acryl-, insbesondere (Meth-)Acrylatgruppen. Eine organische Polymerisation durch Wärme ist gegenüber einer solchen Polymerisation durch Licht überlegen, weil die thermische Härtung technisch sehr einfach realisiert werden kann. Gegebenenfalls kann dies jedoch ungünstig sein, z.B. wenn das Substrat empfindlich auf Wärme reagiert, weshalb auch lichtinduziert polymerisierbare Gruppen geeignet sein können. Die Anwesenheit von Epoxygruppen ist besonders bevorzugt, weil diese nicht nur einer wärmeinduzierten Polymerisation unterworfen werden können, sondern bei der Polymerisation außerdem Hydroxygruppen entstehen, die eine gute Haftung zum Substrat gewährleisten.

Die Komponente, die die Beschichtungseigenschaften positiv beeinflusst, ist vorzugsweise eine anorganische Komponente mit einem organischen Anteil, nämlich ein Silan mit einem über Kohlenstoff an das Silicium gebundenen Rest, der eine organisch polymerisierbare Gruppe trägt, beispielsweise ein Epoxysilan oder ein (Meth-)Acrylsilan. Bewährt hat sich der Einsatz eines alkoxymodifizierten Epoxysilans, z.B. von (3-Glycidyloxypropyl)trimethoxysilan, bekannt unter der Abkürzung GLYMO. Dieses Silan kann zusammen mit dem die biologisch abbaubare Komponente tragenden Silan und ggf. einer oder mehreren hydrolytisch kondensierbaren Verbindungen der Formel M^{b}(X)_{b} einer hydrolytischen Kondensationsreaktion unterworfen werden.

Außerdem ist es wünschenswert, dass die durch Applikation aus dem Lack erhaltenen Schichten gute Barriereeigenschaften bezüglich des Durchtritts von Sauerstoff und Wasserdampf aufweisen. Aus Erfahrung ist bekannt, dass gute Barrierewirkungen gegenüber Sauerstoff und Wasserdampf einhergehen mit Barrierewirkungen auch gegenüber anderen Gasen, Dämpfen und Geruchsstoffen. Dies ist z.B. dann sehr bedeutend, wenn die zu beschichtenden Tabletten vor solchen Gasen, Dämpfen oder Geruchsstoffen geschützt werden sollen oder um die Abgabe von Gerüchen zu verhindern oder zu senken, die von zu beschichtenden Lebensmitteln ausgehen.

Das für die Beschichtung von Lebensmitteln und Tabletten erfindungsgemäß geeignete Beschichtungsmaterial kann wie folgt hergestellt werden:
(a) nach dem Sol-Gel-Verfahren durchgeführte Erzeugung eines Kieselsäure(hetero)polykondensats durch hydrolytische Kondensation von
   (i) mindestens einem Tetraalkylorthosilikat und/oder mindestens einem Glycidyltrialkoxysilan, sowie
   (ii) einem mit einer oder zwei Silylgruppen funktionalisiertem Polycaprolacton-Derivat, vorzugsweise einem Polycaprolacton-triol, das mindestens zwei Komponenten [O-(CH₂)ₘ-C(O)-O]ₙ trägt, worin m = eine ganze Zahl zwischen 2 und 8 ist und n 1, 2 oder 3 oder größer 3 ist, wobei n unterschiedliche Werte annehmen kann,
      wobei das Kieselsäure(hetero)polykondensat weiterhin
   (iii) optional unter Verwendung einer hydrolytisch kondensierbaren Aluminiumverbindung, vorzugsweise einem komplexierten Aluminium-tert.-Butylat, sowie
   (iv) einem Lösungsmittel, ausgewählt unter Alkoholen und acetylacetonatgruppenhaltigen Verbindungen, optional in Mischung mit Wasser,
   hergestellt wurde, und gegebenenfalls
(b) Einarbeiten eines antibakteriell wirksamen Stoffs in das Kieselsäure(hetero)polykondensat.

In besonders günstigen Ausgestaltungen der Erfindung wird das erfindungsgemäße Beschichtungsmaterial in Kombination mit einer anorganischen, vorzugsweise aus der Gasphase abgeschiedenen Schicht wie SiOₓ mit x = 1,5 - 1,8, oder einem anderen Metalloxid eingesetzt. Dabei kann die aus der Gasphase abgeschiedene Schicht direkt auf das Substrat aufgebracht und mit dem erfindungsgemäßen Beschichtungsmaterial überschichtet werden, oder umgekehrt. Besonders günstig sind Schichtkombinationen, in denen eine erfindungsgemäße Schicht über eine aus der Gasphase abgeschiedene Schicht appliziert wird oder zwischen zwei solchen Schichten liegt. Mit den genannten Kombinationen beschichtete Substrate zeigen nochmals verbesserte Barrierewerte, und zwar auch bei relativ dünner hybridpolymerer Schicht (insbesondere unter 10 µm, z.B. im Bereich von 1 bis 5 µm). Die mit einer Kombination aus einer SiOₓ-Schicht und einer erfindungsgemäßen Schicht beschichteten Substrate zeigten Barrierewerte gegenüber dem Durchtritt von Sauerstoff und Wasserdampf, die weit unter den für Lebensmittelverpackungen geforderten Werten lagen.

Die voranstehend beschriebenen Lacke lassen sich auf die Medikamentenkerne bzw. Lebensmittel applizieren. Der Auftrag kann über verschiedenste Applikationstechniken erfolgen (beispielsweise durch Sprühen). Nach der organischen Vernetzung des Lackes werden so Barrierebeschichtungen für die Tabletten bzw. Lebensmittel erhalten.

Die Auftragsmenge liegt vorzugsweise zwischen 3 und 10 Massen-%. Die Schichtdicke kann vorzugsweise auf Bereiche zwischen 1 bis 250 µm, stärker bevorzugt auf 5 bis 100 µm, eingestellt werden.

Aus dem Stand der Technik ist außerdem die Einarbeitung von inerten Füllstoffen in die Beschichtung bekannt. Diese sollten nicht chemisch reaktiv sein, nicht hygroskopisch, dispergierbar und eine Partikelgröße haben, welche das optische Erscheinungsbild der Beschichtung nicht beeinflusst. Typische Füllstoffe sind Stärke, chemisch modifizierte Stärke, Dextrin, mikrokristalline Cellulose, unlösliche Cellulosederivate, sowie anorganische Verbindungen (z.B. Talk, TiO₂, SiO₂, Silikate, Tonmaterialien, unlösliche Carbonate und Phosphate). Der Anteil des Füllstoffes (vorzugsweise in einer Menge von 1 - 25% der Beschichtung) ist abhängig vom Material. Stärke oder Dextrin verbessern die mechanischen Eigenschaften und erleichtern die Verarbeitbarkeit. Anorganische Füllstoffe wie Silikate verbessern die Feuchtebarriere.

Erfindungsgemäß zeigt sich, dass die Kieselsäure(hetero)polykondensate mit eingearbeitetem PCL-T eine gute Haftung auf verschiedenen Substraten besitzen und transparent sowie biologisch abbaubar sind.

### Ausführungsbeispiele

### Verwendete Materialien:

Epoxyfunktionalisiertes Alkoxysilan (GLYMO), zwei weitere Metallalkoholate (Aluminium-tri-sec. Butoxid, AsB und ein Tetraalkoxysilan, meist Tetramethoxysilan), Ammoniumchlorid, Ammoniumhydroxid, Ethanol, Komplexierungsmittel (Ethylacetoacetat, EAA), Isocyanathaltiges Alkoxysilan (Isocyanatopropyltriethoxysilan), Polycaprolacton-triol, Salzsäure 0,1 M.

### A1 - Funktionalisierung von Polycaprolaton-triol mit Triethoxysilangruppen

Die Funktionalisierung von Polycaprolacton-triol (PCL-T) mit Triethoxysilangruppen erfolgte angelehnt an Liu *et al.* (LIU, P.; HE, L.; SONG, J.; LIANG, X.; DING, H.: Microstructure and thermal properties of silylterminated polycaprolactone-polysiloxane modified epoxy resin composites. In: Journal of Applied Polymer Science 109 (2008), S. 1105-1113). Zuerst wurden 50,0 g (166,67 mmol) PCL-T getrocknet, um absorbiertes Wasser zu entfernen. Die Trocknung erfolgte unter Vakuum (Drehschieberpumpe) bei 100°C. Die Funktionalisierung von einem Drittel (bzw., in der Alternative, von zwei Dritteln) der Hydroxygruppen des PCL-T wurde anschließend bei 80°C unter Argon durchgeführt, indem 41,23 g bzw. 166,67 mmol (42,46 g bzw. 333,34 mmol) Isocyanatopropyltriethoxysilan innerhalb einer Stunde zum PCL-T zugetropft wurden. Die Reaktionsmischung wurde für 18 h bei 80°C unter Argon gerührt. Die Reaktion wurde mittels IR-Transmissionsspektroskopie auf Vollständigkeit überprüft. Eine breite Bande zwischen 3750 und 3000 cm⁻¹, die den Schwingungen der Hydroxygruppen des PCL-T zuzuordnen ist, bleibt erhalten, was zeigt, dass während der Funktionalisierung des PCL-T keine Hydrolyse der Triethoxysilangruppen stattfindet. Eine dominante Bande der NCO-Gruppe bei 2272 cm⁻¹ war nach 18 Stunden vollständig verschwunden, was bestätigt, dass alle Isocyanatogruppen des Isocyanatopropyltriethoxysilans unter Bildung einer Carbamatgruppe umgesetzt waren. Es wurde eine klare, hochviskose Flüssigkeit erhalten. Die Charakterisierung des Produkts wurde mittels IR-Transmissions- (Nicolet 6700, Firma Thermo Fisher Scientific, Auflösung: 4cm⁻¹, Messbereich: 4000 cm⁻¹ bis 400 cm⁻¹) und NMR-Spektroskopie (DPX400, Bruker) durchgeführt. Über die Berechnung der Integrale im ¹H-NMR-Spektrum konnte nachgewiesen werden, dass die Einfach-Funktionalisierung zu 100% des einfach funktionalisierten Produkts führte und die zweifache Funktionalisierung eine fast ebenso hohe Ausbeute ergab.

### A2 - Funktionalisierung von Cellulose mit Epichlorhydrin und anschließende Vernetzung mit einem epoxygruppenhaltigen Kieselsäure(hetero)polykondensat

Für die Funktionalisierung wurden nach Dong et al [Dong, S. ; Roman, M.; J. Am. Chem. Soc. 129 (2007), 13810] 4,00 g (24,66mmol) Cellulose in 328 ml einer 1 M Natronlauge dispergiert und anschließend mit 2,28 g (24,7 mmol) Epichlorhydrin für 2 h bei 60°C gerührt. Das erhaltene weiße, kreidige Pulver wurde abfiltriert und mit destilliertem Wasser bis zur Neutralität gewaschen. Es wurde bei 60°C getrocknet und fein zermörsert.

Anschließend wurde ein nach dem Stand der Technik hergestelltes epoxygruppenhaltiges Kieselsäurepolykondensat mit der funktionalisierten Cellulose vermischt; die Mischung wurde einer Polymerisationsreaktion unterworfen.

### B - Herstellung und Charakterisierung der Lacksysteme

| Bezeichnung der Beispiele | Masseanteil des funktionalisierten PCL-T [Gew.-%] | Veränderung der Lackrezeptur zum Referenzlack |
|---|---|---|
| 1-PCL-T-1/3 | 16 | Referenzlack |
| 2-PCL-T-1/3 | 29 | Erhöhung des PCL-T-Anteils im Lacks auf Kosten von GLYMO |
| 3-PCL-T-1/3 | 41 | Erhöhung des PCL-T-Anteils im Lacks auf Kosten von GLYMO |
| 1-PCL-T-2/3 | 16 | Erhöhung des Funktionalisierungsgrads des PCL-T |
| 1-PCL-T-1/3-GLYMO | 16 | Erhöhung des GLYMO-Anteils im Lack auf Kosten von TMOS |
| 1-PCL-T-1/3-TMOS | 16 | Erhöhung des TMOS-Anteils im Lack auf Kosten von GLYMO |

### Vergleichsbeispiel:

Es wurde ein Standardlack unter Verwendung der folgenden Komponenten hergestellt:
GLYMO, vorzugsweise in einer Menge von 40-80 Mol-%, bezogen auf alle siliciumhaltigen Ausgangsmaterialien,
TMOS vorzugsweise in einer Menge von 20-60 Mol-%, bezogen auf alle siliciumhaltigen Ausgangsmaterialien,
mit Ethylacetoacetat komplexiertes Aluminium-tert.-Butylat, vorzugsweise in einer Menge von 1 und 30 Mol-%, bezogen auf die molare Menge an GLYMO plus TMOS.

Die Komplexierung des Aluminium-tert.-Butylats dient dazu, die Hydrolyse und Kondensation der Aluminiumverbindung zu verlangsamen, um ein zu schnelles Wachstum des Oxidnetzwerkes zu verhindern.

Für die Synthese des Standardlacks wurde AsB vorgelegt und EAA zugetropft. Nachdem die Mischung 30 min gerührt worden war, wurden TMOS, GLYMO zum Ansatz zugegeben und homogen vermischt. Anschließend wurde Wasser zugetropft (1-stöchiometrisch zu den hydrolysierbaren Gruppen) und der Lackansatz 3 h lang bei RT gerührt. Es wurde ein klares farbloses Sol mit einem Feststoffgehalt von 49 Gew.-% erhalten.

### Beispiel "Referenzlack" (1-PCL-T-1/3):

Das Vergleichsbeispiel wurde mit der Maßgabe wiederholt, dass ein Teil des GLYMO durch Sifunktionalisiertes PCL-T (ein Drittel der Hydroxygruppen ersetzt) ersetzt wurde derart, dass der Masseanteil des funktionalisierten PCL-T (feststoffbezogen) bei 16 Gew.-% lag.

Alle weiteren Lacksysteme wurden ausgehend von der oben beschriebenen Synthese mit Veränderungen in der Lackrezeptur hergestellt. Alle durchgeführten Änderungen sind in der voranstehenden Tabelle aufgeführt. An der grundsätzlichen Vorgehensweise bei der Synthese wurden keine Veränderungen vorgenommen.

Vor der sich anschließenden Applikation der Lacke wurden alle Systeme mit einem Ethanol/Wasser-Gemisch (Masseverhältnis 1:1) auf einen Feststoffgehalt von 22 Gew.-% (exklusive des antibakteriellen Additivs) verdünnt.

Die Vollständigkeit der Hydrolysereaktion nach der Lacksynthese wurde mittels Raman-Spektroskopie (Spektrometer RFS 100 der Firma Bruker, Leistung: 500 mW, Messbereich: 4000 cm⁻¹ bis 200 cm⁻¹, Auflösung: 4 cm⁻¹) untersucht.

Die Haltbarkeit der Lacksysteme wurde sowohl mittels Raman-Spektroskopie als auch der Bestimmung der kinematischen Viskosität (Ubbelohdeviskosimeter Typ AVS 410 der Schott/SI Analytics GmbH) überprüft.

Die Ermittlung des Feststoffgehalts der Lacksysteme erfolgte mittels Ausheizen bei 200°C für eine Stunde. Es wurde jeweils eine Doppelbestimmung durchgeführt.

### Rakelbeschichtung und Aushärtung

Die Beschichtung der Lacke wurde mittels Spiral-Rakel (Modell 358) von Erichsen und dem Filmziehgerät Coatmaster 509 MC von Erichsen durchgeführt. Es wurde mit einem Spiral-Rakel mit einer Filmbreite von 220 mm und einer Drahtwicklung von 20 µm gearbeitet. Die entstandenen Nassfilmdicken lagen somit im Bereich von 20 µm. Die Applikationsgeschwindigkeit betrug 12,5 mm/s. Als Substrat wurde PLA-Folie (30 µm) bzw.

Cellophan-Folie (45 µm, 20x27 cm) und für die Festkörper-NMR-Proben eine Teflonplatte verwendet. Für die Messungen des Bioabbaus wurde PET-Folie (100 µm) eingesetzt. Bei der Überprüfung der Schichthaftung wurden die Substrate mit Corona vorbehandelt. Die Aushärtung der Beschichtungen erfolgte bei 100°C für 1 h (PLA-Folien) bzw. 130°C für 1 h (PET-Folien und Teflonplatte) in einem Konvektionsofen U 80 der Firma Memmert GmbH + Co. KG. Nach dem Aushärten wurde eine Dicke der Beschichtung von ca. 5 µm gemessen.

Vergleichbar dicke Beschichtungen lassen sich mit für Tablettenbeschichtungen übliche Verfahren erreichen.

### Beurteilung von Optik und Haftung

Die entstandenen Schichten wurden hinsichtlich ihrer Optik und ihrer Haftung auf Polylactat-(PLA) und Polyethylentherephtalat- (PET) Folien untersucht. Die optische Beschaffenheit wurde mit bloßem Auge beurteilt. Die Haftung wurde mittels Tape-Test ermittelt, welcher sich von der Gitterschnitt-Technik mit Klebeabriss ableitet (DIN EN ISO 2409). Die Haftung der Schicht auf dem Substrat wird dabei mit den Kategorien TT 0 (die Schicht wird durch den Test nicht vom Substrat gelöst) bis TT 5 (die Schicht wird vollständig vom Substrat gelöst) beurteilt. Für den Test wurde ein Klebeband mit definierter Klebkraft blasenfrei auf die Beschichtung geklebt und angepresst. Nach einer Dauer von 1 min wurde das Klebeband innerhalb einer Sekunde in einem Winkel von ca. 60° zur Zugrichtung abgezogen und anschließend die Haftung bewertet.

Mit allen Materialien wurde eine transparente, glatte, homogene, defektfreie Beschichtung erhalten. Unabhängig vom gewählten PCL-T-Gehalt sowie den weiteren Variationen der Lackzusammensetzung hatten alle Schichten die gleiche, gute, Qualität. Der Tape-Test ergab die folgenden Werte:

| | PLA | | PET | |
|---|---|---|---|---|
| bioORMOCER® | ohne Corona | mit Corona | ohne Corona | mit Corona |
| 1-PCL-T-1/3 | TT 5 | TT 0 | TT 1 | TT 0 |
| 2-PCL-T-1/3 | TT 5 | TT 1 | TT 1 | TT 0 |
| 3-PCL-T-1/3 | TT 5 | TT 2 | TT 2 | TT 0 |
| 1-PCL-T-1/3-TMOS | TT 5 | TT 1 | TT 1 | TT 0 |
| 1-PCL-T-1/3-GLYMO | TT 5 | TT 0 | TT 0 | TT 0 |
| 1-PCL-T-2/3 | TT 5 | TT 0 | TT 0 | TT 0 |

### Biologische Abbaubarkeit

Die biologische Abbaubarkeit der hergestellten Beschichtungen wurde nach Norm ISO 14885-1:2005 und durch Lagerung der Proben in einem Kompost getestet. Die beschichteten PET-Folien wurden für den Komposttest in einen Dia-Rahmen geklemmt und rückseitig mit einer nicht abbaubaren Kontrollprobe (Beschichtung auf PET-Folie) verbunden. Die Proben wurden danach senkrecht im Kompost platziert, so dass die gesamte Probe von Erde bedeckt war. Der Kompost wurde aus sogenanntem trockenen Aktivkompost der Kompostwerke Würzburg GmbH, Wasser (ca. 50 Gew.-% bezogen auf den trockenen Kompost) und Radivit® Kompostbeschleuniger der Firma Neudorff (ca. 5 g pro Liter Kompost) hergestellt. Die Mikroorganismen des Komposts und des Kompostbeschleunigers sind so ca. zwei bis drei Wochen lang aktiv. Der Kompost wurde alle 3 Wochen gewechselt und alle Proben vorsichtig gesäubert und fotografiert. Bei sichtbaren Anzeichen für einen biologischen Abbau wurden die Proben mittels Laser-Scanning-Mikroskopie (VK-X200 von Keyence Corporation) untersucht.

Die Lagerung der Beschichtungen aus 1-PCL-T-1/3, 2-PCL-T-1/3 und 3-PCL-T-1/3 im Kompost führte zu ersten Abbauerscheinungen, die umso stärker waren, je höher der PCL-T-Anteil im Material war.

Die biologische Abbaubarkeit mit Hilfe von in Kompost enthaltenen Mikroorganismen ist ein Indikator dafür, dass die Schichtmaterialien auch von höheren Organismen, darunter Säuretieren und Menschen, oder von deren Darmbakterien, zersetzt werden.

### Netzwerkbildung

Die anorganische Netzwerkdichte der ausgehärteten Lacksysteme wurde mittels ²⁹Si-Festkörper-HPDEC/MAS-NMR-Spektroskopie untersucht. Über die Zuordnung der T- und Q-Gruppen konnte sichergestellt werden, dass alle beteiligten Präkursoren in das anorganische Netzwerk eingebunden wurden.

Die anorganische Netzwerkdichte konnte anhand der Intensitätsverhältnisse der T-Gruppen abgeschätzt werden. Tabelle 1 zeigt die Netzwerkdichte der verschiedenen Lacksysteme im Vergleich.

**Tab. 1**

| **anorganische** Netzwerkdichte von | | **anorganische** Netzwerkdichte von | | anorganische Netzwerkdichte von |
|---|---|---|---|---|
| 2-PCL-T-1/3 | < | 1-PCL-T-1/3 | < | 1-PCL-T-1/3-GLYMO |
| 1-PCL-T-1/3-TMOS | | | | 1-PCL-T-2/3 |

In der nachstehenden Tabelle 2 wird die Vernetzungsdichte der organischen und anorganischen Netzwerkstruktur der ausgehärteten Schichtsysteme auf Basis der Auswertung der ¹³C-Festkörper-NMR- und ²⁹Si-Festkörper-NMR-Spektren in Bezug auf das Referenzsystem 1-PCL-T-1/3 eingeordnet.

**Tab. 2**

| **Lacksystem** | **Vergleich der Vernetzungsdichte des anorganischen Netzwerks** | **Vergleich der Vernetzungsdichte des organisches Netzwerks** |
|---|---|---|
| 2-PCL-T-1/3 | Offener | etwas dichter |
| 1-PCL-T-1/3-TMOS | Offener | 0 |
| 1-PCL-T-1/3 **(Referenzsystem)** | **0** | **0** |
| 1-PCL-T-1/3-GLYMO | Dichter | 0 |
| 1-PCL-T-2/3 | Dichter | etwas offener |

### Barriereeigenschaften

Mittels PVD (Physikalische Gasphasenabscheidungen) aufgebrachte SiOₓ-Schichten auf Polyethylenterephthalat (PET/SiOₓ) besitzen Wasserdampf-Permeabilitäten (WVTR) im Bereich von 0,12 g/m²/d. Ihre Sauerstoffdurchlässigkeit (OTR) liegt bei etwa 0,23 cm³/m²/d/bar. Diese Werte liegen etwas über den für Lebensmittelverpackungen geforderten Werten von 0,10 g/m²/d für die WVTR und von 0,1 cm³/m²/d/bar für die OTR. Wird diese anorganische Schicht mit einer "klassischen" Hybridschicht aus organisch modifizierten

Kieselsäure(hetero)polykondensaten wie dem oben beschriebenen "Standardlack" kombiniert, lassen sich diese Werte bei Schichtdicken von ca. 1 bis 5 µm um bis zu 92% (WVTR) bzw. 96% (OTR) und damit auf für Lebensmittelverpackungen geforderte Werte verringern. Für eine Kombination einer SiOₓ-Schicht mit jeweils einer der erfindungsgemäßen Schichten konnten bei vergleichbaren Schichtdicken Werte ermittelt werden, die immerhin noch um 69-85% (WVTR) bzw. 87-91 % (OTR) gegenüber dem System PET/SiO₂ verringert waren. Dies zeigt, dass der Einbau von biologisch abbaubaren Komponenten in das Kieselsäure(hetero)polykondensat den Schichten nur wenig von ihren hervorragenden Barriereeigenschaften nimmt. Die geringe Wasserdampftransmissionsrate ist dabei besonders erstaunlich.

Dementsprechend eignen sich die erfindungsgemäßen Lacke zur Herstellung von Beschichtungsmaterialien für Lebensmittel und pharmazeutische Produkte.

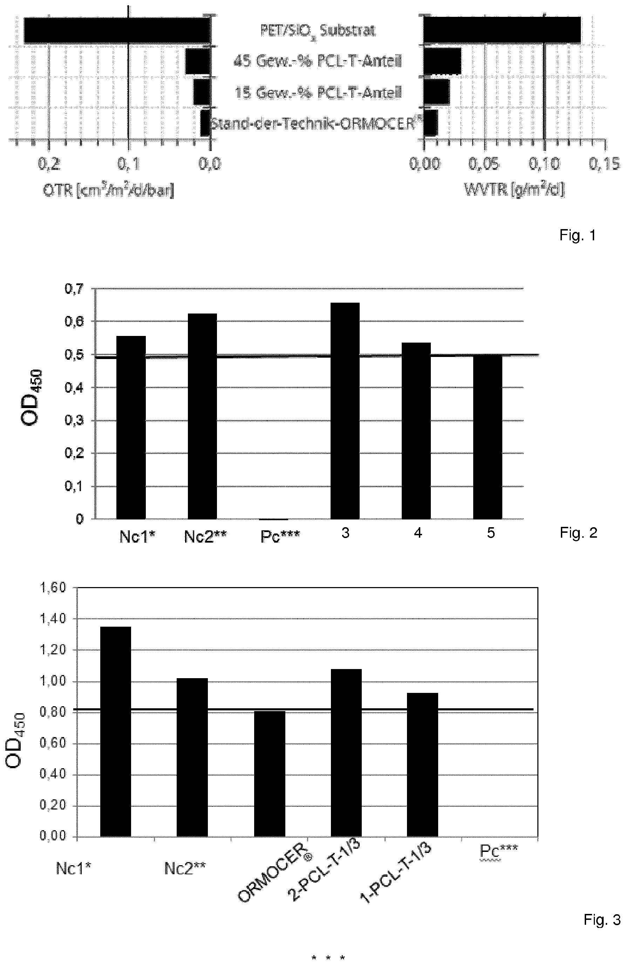

Die beigefügte Figur 1 zeigt die ermittelten Werte. Als "Stand-der-Technik-ORMOCER®" wird hier ein Lack bezeichnet, der aus einem Hybridmaterial gemäß dem oben beschriebenen Vergleichsbeispiel hergestellt war. Die Beschichtung, die mit "45 Gew.-% PCL-T-Gehalt" bezeichnet wird, entspricht in etwa dem obigen Beispiel 3-PCL-T-1/3, wobei jedoch ein noch etwas größerer Anteil von GLYMO gegen PCL-T-ausgetauscht worden war, derart, dass der Gehalt an PCL-T von 41 auf 45 Gew.-% gesteigert wurde. Die Beschichtung, die mit 15 Gew.-% PCL-T-Gehalt" bezeichnet wird, entspricht in etwa dem obigen Beispiel 1-PCL-T-1/3, also dem Referenzlack, wobei jedoch etwas weniger GLYMO durch PCL-T ersetzt wurde, so dass dessen Gehalt nur 15 Gew.-% beträgt.

Für die Verwendung als Tabletten- bzw. Lebensmittelbeschichtung ist eine makellose Optik notwendig. Es wurde die Optik von zwei entwickelten Beschichtungen auf einem Mustersubstrat auf Polyethylenterephthalat-Basis (PET-Substrat) untersucht. Die entwickelten biopolymerhaltigen Schichten wiesen entweder 30 Gew.-% Chitosan-Anteil oder 45 Gew.-% PCL-T-Anteil auf einem PET-Substrat auf. Bei beiden hergestellten PCL-T- bzw. chitosanhaltigen Beschichtungen wurde eine transparente, glatte, homogene Oberfläche erhalten, die keine Defekte zeigte. Unabhängig vom gewählten Biopolymer-Gehalt wiesen die Schichten, bei einer hervorragenden Haftung auf dem Substrat, die gleiche gute optische Qualität auf.

### Biologisch abbaubare Schichten mit guten Barriereeigenschaften

Tablettenbeschichtungen benötigen insbesondere dann außerordentlich gute Barriereeigenschaften gegenüber Geruchsstoffen, Wasserdampf und Sauerstoff, wenn es sich um Sauerstoff- bzw. Wasserdampf-empfindliche Wirkstoffe handelt. Durch die Anpassung der Netzwerkdichte und der Polarität des hybridpolymeren Beschichtungsmaterials ist es in Kombination mit anorganischen Oxidschichten, wie z. B. SiOx (1,5 < x < 1,8), möglich, sehr gute Barrierewerte zu erzielen [Amberg-Schwab, S.: Handbook of Sol-Gel Science and Technology, Vol. 3, Ed.: S. Sakka, Kluwer Academic Publishers, Norwell, Chap.21 (2004), S. 455]. Die aufgebrachte Hybridpolymerschicht reduziert die Mikro- und Nanoporosität.

Die Kombination mit einer anorganischen Oxidschicht kann eine funktionelle Beschichtung darstellen. Das erfindungsgemäß verwendete Material kann in einem Schichtverbund mit einer oder mehreren anderen, vorzugsweise anorganischen Schichten, vorliegen. Der Schichtverbund ist essbar.

Es wurden beispielhaft für die Erfindung die Sauerstoffpermeabilität und die Wasserdampfpermeabilität, jeweils mit und ohne Hybridpolymer-Barrierebeschichtung, untersucht. Die Untersuchung der Sauerstoffpermeabilität erfolgte nach DIN 53 380 (23°C, 50% r. h., relative Feuchtigkeit), die Untersuchung der Wasserdampfpermeabilität nach DIN 53 122 (23°C, 85% r. h., relative Feuchtigkeit). Es ergab sich für das Substrat eine Sauerstoffpermeabilität von 160 cm³/m²_{*}d_{*}bar, für das mit Hybridpolymer beschichtete Substrat eine Sauerstoffpermeabilität von 10 cm³/m²_{*}d_{*}bar. Bei der Wasserdampfpermeabilität ergab sich für das bioabbaubare Modellsubstrat ein Wert von 140 g/m²_{*}d, für das mit Hybridpolymer beschichtete bioabbaubare Modellsubstrat ein Wert von 30 g/m²_{*}d.

Figur 1 zeigt die Sauerstoff- (OTR, bei 50 % r. h. und 23°C) und Wasserdampftransmissionsrate (WVTR, bei 90% r. h. und 38°C) der entwickelten Chitosan- und PCL-T-haltigen Beschichtungen in einem Schichtsystem aus PET-Folie und SiOₓ-Schicht. Die Marke bei 0,1 cm³/m²_{*}d_{*}bar bei der Sauerstoffpermeabilität und die Marke bei 0,10 g/m²_{*}d bei der Wasserdampfpermeabilität stellt die Anforderung bezüglich der Barriere an Lebensmittelverpackungen dar.

### Cytotoxizitäts-Untersuchungen

In den Figuren 2 und 3 ist die in vitro Cytotoxizität einiger PCL-T-Materialien angegeben Figur 2: In vitro Zytotoxizität von mit dem erfindungsgemäß einsetzbaren Lack beschichteten Glasplättchen

Zellen der Murine Fibroblasten-Zelllinie (L929) wuchsen für 24h auf drei mit verschiedenen organisch modifizierten Kieselsäurepolykondensaten beschichteten Glasplatten. Nachdem das Tetrazolium-enthaltende Reagenz WST-1 hinzugegeben wurde, wurde die optische Dichte (OD₄₅₀) der Zellkultur gemessen. 3 stellt das Ergebnis für die Glasplatte mit Referenzlack dar, 4 zeigt das Ergebnis für die Glasplatte mit 2-PCL-T-1/3 und 3 das Ergebnis für die Glasplatte mit 1-PCL-T-1/3.

In der Figur bedeuten:
* = Zellen wurden auf well bottoms (im Gefäß, ohne Glassubstrat) aufgewachsen
** = Zellen wurden auf die Glasplatten aufgewachsen
*** = Zellen wurden auf well bottoms aufgewachsen und toxisch behandelt mit 1% SDS (das heißt gezielt getötet, um eine toxisches Substrat zu imitieren) (SDS = Natriumdodecylsulfat)

Da die L929 Lebensfähigkeit proportional zum Wert der optischen Dichte der Zellkultur ist, lässt sich aus OD450 die Zytotoxizität bestimmen: Eine Probe gilt dann als nicht zytotoxisch, wenn OD₄₅₀ ≥ 80% des Wertes für die Blindprobe Nc2 beträgt (die schwarze Linie kennzeichnet diesen Bereich).

Figur 3: In vitro Zytotoxizität von mit dem erfindungsgemäß einsetzbaren Lack beschichteten Glasplättchen

Kieselsäurepolykondensat-Extrakte wurden erhalten durch Schütteln der sterilen mit dem Kieselsäurepolykondensat beschichteten Glasplatten für 24h in einem Zellkulturmedium (DMEM - Dulbecco's Modified Eagle Medium) unter physiologischen und aseptischen Bedingungen. Anschließend wurden die subkonfluente Zellkulturen (L929) für 24h in unverdünnten Extraktionslösungen inkubiert und analog zur vorherigen Folie präpariert, um OD₄₅₀ zu ermitteln.

Mit "Ormocer" ist der Referenzlack bezeichnet.

In der Figur bedeuten:
* = Zellen wurden auf well bottoms (im Gefäß, ohne Glassubstrat) aufgewachsen
** = Zellen wurden im Medium, welches wie die Extrakte 24h lang inkubiert war, wachsen gelassen
*** = Zellen wurden auf well bottoms aufgewachsen und toxisch behandelt mit 1% SDS (das heißt gezielt getötet, um eine toxisches Substrat zu imitieren) (SDS = Natriumdodecylsulfat)

Die Figuren zeigen, dass die beiden erfindungsgemäß einsetzbaren Beschichtungsmaterialien gegenüber Säuretierzellen nicht zytotoxisch sind, da sie den Minimalwert erreichen. Deshalb können diese Zusammensetzungen als Beschichtungen für Arzneimittel und Lebensmittel verwendet werden.

Extrakt aus Kontakt mit organisch modifiziertem Kieselsäurepolykondensat ohne biologisch abbaubare Gruppen ist schwach zytotoxisch.

Durch eine biologisch degradierbare, gesundheitlich unbedenkliche Beschichtung auf den Tabletten, die hervorragende Barriereeigenschaften haben, ist es möglich, PVDC und Aluminium durch alternative Verpackungsmaterialien zu ersetzen, die selbst keine herausragende Barrierewirkung besitzen müssen, aber tiefziehfähig sind. Darüber hinaus sind auch alternative, einfachere Verpackungskonzepte denkbar, beispielsweise ein Verzicht auf die Vereinzelung in Blisterverpackungen.

Die Eigenschaften der hochfunktionellen Beschichtungsmaterialien lassen sich über die Barrierewirkung hinaus gezielt einstellen, kombinieren und maßschneidern. Wie oben erläutert, sind die Beschichtungen beispielsweise stabil im sauren Milieu, können aber auch so eingestellt werden, dass sie sich bereits im Magen auflösen. Die Zusammensetzung der Beschichtung kann so ausgewählt werden, dass sie sich im Körper unbedenklich verhält.

Durch die exzellenten Barriereeigenschaften der neu entwickelten Beschichtung können zukünftig bei den Verpackungsmaterialien chlorhaltige Folien und Aluminiumfolien eingespart werden. Alternative, günstigere Verpackungskonzepte sind denkbar. Die Barrierebeschichtung verbessert darüber hinaus die Haltbarkeit der Pharmaprodukte und Lebensmittel.

Außerdem können durch die erfindungsgemäße Verwendung Produkte hergestellt werden, die essbare Beschichtungen sind und folgenden Eigenschaften haben:
- Keine toxischen, allergenen und nicht-verdaubaren Substanzen
- Strukturelle Stabilität und mechanische Beschädigungen während des Transports, Handlings und Verkaufs verhindern
- Gute Adhäsion an der Lebensmitteloberfläche und gleichmäßige Beschichtung
- Semipermeabel: Für einige Produkte ist eine kontrollierte Gas- bzw. Wassermigration in und aus dem zu schützenden Produkt wünschenswert. Beispiel hierfür ist der Obst- und Gemüsetransport, da die Produkte oft noch während des Transports zum Handel einem Reifeprozess unterliegen (aerobe und anaerobe Atmung).
- Verhinderung der Aufnahme oder Abgabe von Komponenten, welche das Aroma, den Geschmack, Nährstoffe und die organoleptische Eigenschaften stabilisieren; ohne dabei den Geschmack oder das optische Erscheinungsbild zu verändern
- Gewährleisten einer biochemischen und mikrobiellen Stabilität und gleichzeitig schützen gegen Verunreinigungen, Schädlingsbefall, Mikrobenausbreitung und weitere Arten des Zerfalls
- Erhalten oder Verbessern der ästhetischen und sensorischen Attribute (Aussehen, Geschmack, etc.) des Lebensmittels
- Einsatz als Carrier für gewünschte Additive wie: Geschmack, Geruch, Farbe, Nährstoffe und Vitamine. Einbau von Antioxidantien und antimikrobiellen Wirkstoffen.
- Einfache und kostengünstige Herstellung

## Patentansprüche

1. Verwendung einer Zusammensetzung, die mit organischen Gruppen modifiziertes Kieselsäure(hetero)polykondensat enthält, als auf die Oberfläche eines unter einem Arzneimittel und einem Lebensmittel ausgewählten Substrats aufgetragene Beschichtung, oder als Komponente einer solchen Beschichtung, wobei die organischen Gruppen Polymere sind.

2. Verwendung nach dem vorstehenden Anspruch, wobei die organischen Gruppen eine oder mehrere Arten von natürlichen oder synthetischen organischen Polymeren sind.

3. Verwendung nach Anspruch 1 oder 2, wobei die organischen Gruppen teilweise oder ausschließlich biologisch abbaubare Gruppen sind.

4. Verwendung nach einem der vorstehenden Ansprüche, wobei die organischen Gruppen zumindest teilweise über Si-C-Bindungen in das Kondensat eingebunden vorliegen.

5. Verwendung nach einem der vorstehenden Ansprüche, worin das Kieselsäure(hetero)polykondensat weiterhin über Si-C-Bindungen in das Kondensat eingebundene organische Gruppen aufweist, die thermisch oder photochemisch organisch polymerisierbar sind, insbesondere Epoxygruppen.

6. Verwendung nach einem der vorstehenden Ansprüche, wobei zumindest ein Teil der organischen Gruppen eine Kohlenwasserstoffkette mit 2 bis 8, vorzugsweise mit 2 bis 6 Kohlenstoffatomen aufweist, die sich zwischen zwei unter Ether-, Ester-, Amid-, und Urethangruppen ausgewählten Gruppen befindet.

7. Verwendung nach einem der vorstehenden Ansprüche, wobei zumindest ein Teil der organischen Gruppen mindestens zwei Kohlenwasserstoffketten mit 2 bis 8 Kohlenstoffatomen aufweist, die verzweigtkettig zueinander vorliegen.

8. Verwendung nach einem der Ansprüche 6 und 7, worin zumindest ein Teil der anorganischen, biologisch abbaubaren Gruppen die Komponente [O-(CH₂)ₘ-C(O)-O]n aufweist, worin m eine ganze Zahl zwischen 2 und 8 ist, n 0, 1, 2 oder 3 oder größer 3 ist und, sofern die genannte Komponente in der organischen, biologisch abbaubaren Gruppe mehrfach vorhanden ist, unterschiedliche Werte annehmen kann, mit der Maßgabe, dass n in der einzigen oder in mindestens einer der genannten Komponenten 1 oder größer 1 ist.

9. Verwendung nach einem der vorstehenden Ansprüche, wobei die organischen Gruppen aus der Gruppe ausgewählt sind, die aus Polycaprolacton-triol, Chitosan, Cellulose und Cellulosebausteinen besteht.

10. Verfahren zur Herstellung eines Beschichtungsprodukts, ausgewählt unter beschichteten Arzneimitteln und Lebensmitteln, welches folgende Stufen umfasst:
(i) das Auftragen einer Zusammensetzung, die optional in einem Verdünnungs-und/oder Lösungsmittel vorliegt und mit organischen Gruppen modifiziertes Kieselsäure(hetero)polykondensat enthält, wobei die organischen Gruppen Polymere sind, auf ein unter einem Arzneimittel und Lebensmittel ausgewähltes Substrat;
(ii) das Trocknen und/oder das Aushärten der Zusammensetzung.

11. Verfahren nach Anspruch 10, das vor der Stufe (i) oder nach der Stufe (ii) die Stufe (iii) umfasst, in der das Substrat zumindest teilweise mit einer Metalloxidschicht überzogen wird.

12. Verfahren nach Anspruch 10 oder 11, wobei die in einem der Ansprüche 1 bis 9 beschriebene Zusammensetzung verwendet wird.

13. Beschichtungsprodukt, herstellbar nach dem Verfahren der Ansprüche 10 bis 12.

14. Beschichtungsprodukt, das ein unter einem Arzneimittel und Lebensmittel ausgewähltes Substrat und eine auf das Substrat aufgebrachte Beschichtung einer Zusammensetzung aufweist, die mit organischen Gruppen modifiziertes Kieselsäure(hetero)polykondensat enthält, wobei die organischen Gruppen Polymere sind.

15. Beschichtungsprodukt nach Anspruch 14, worin die in einem der Ansprüche 1 bis 9 angegebene Zusammensetzung enthalten ist.

## Claims

1. A use of a composition containing silica (hetero)polycondensate modified with organic groups as a coating applied to the surface of a substrate selected from a drug and a foodstuff, or as a component of such a coating, wherein the organic groups are polymers.

2. The use according to any of the foregoing claims, wherein the organic groups are one or more types of natural or synthetic organic polymers.

3. The use according to claim 1 or 2, wherein the organic groups are partially or exclusively biodegradable groups.

4. The use according to one of the above claims, wherein the organic groups are at least partially incorporated in the condensate via Si-C bonds.

5. The use according to one of the above claims, wherein the silicic acid (hetero)polycondensate also has organic groups which are incorporated into the condensate via Si-C bonds and which can be polymerised thermally or photochemically organically, in particular epoxy groups.

6. The use according to one of the preceding claims, wherein at least a part of the organic groups comprising a hydrocarbon chain have from 2 to 8, preferably from 2 to 6, carbon atoms located between two groups selected from ether, ester, amide and urethane groups.

7. The use according to one of the above claims, wherein at least a part of the organic groups comprises at least two hydrocarbon chains with 2 to 8 carbon atoms which are branched-chain to one another.

8. The use according to one of claims 6 and 7, wherein at least some of the inorganic, biodegradable groups have the component [O-(CH₂)ₘ⁻C(O)-O]n, wherein m is an integer between 2 and 8, n is 0, 1, 2 or 3 or greater than 3 and, if said component is present several times in the organic, biodegradable group, can assume different values, with the proviso that n is 1 or greater than 1 in the only or in at least one of said components.

9. The use according to any of the foregoing claims, wherein the organic groups are selected from the group consisting of polycaprolactone-triol, chitosan, cellulose and cellulose building blocks.

10. A process for the preparation of a coating product selected from coated pharmaceuticals and foodstuffs comprising the following steps:
(i) applying a composition, optionally in a diluent and/or solvent, containing silica (hetero)polycondensate modified with organic groups, to a substrate selected from a drug and foodstuff;
(ii) drying and/or curing the composition.

11. The process according to claim 10, which comprises, before step (i) or after step (ii), step (iii) in which the substrate is at least partially coated with a metal oxide layer.

12. The process according to claim 10 or 11, using the composition described in one of claims 1 to 9.

13. A coating product producible according to one of claims 10 to 12.

14. A coating product comprising a substrate selected from a medicament and foodstuff and a coating applied to the substrate of a composition comprising silica (hetero)polycondensate modified with organic groups.

15. The coating product according to claim 14, in which the composition indicated in one of claims 1 to 9 is contained.

## Revendications

1. Utilisation d'une composition, qui contient un (hétéro)polycondensat d'acide silicique modifié avec des groupes organiques, en tant qu'enrobage appliqué sur la surface d'un substrat choisi parmi des produits pharmaceutiques et alimentaires, ou en tant que constituant d'un tel enrobage, les groupes organiques étant des polymères.

2. Utilisation selon la revendication précédente, dans laquelle les groupes organiques sont un ou plusieurs types de polymères organiques naturels ou synthétiques.

3. Utilisation selon la revendication 1 ou 2, dans laquelle les groupes organiques sont des groupes partiellement ou exclusivement biodégradables.

4. Utilisation selon une des revendications précédentes, dans laquelle les groupes organiques sont présents dans le condensat et liés au moins partiellement par des liaisons Si-C.

5. Utilisation selon une des revendications précédentes, dans laquelle le (hétéro)polycondensat d'acide silicique présente en outre des groupes organiques liés par des liaisons Si-C dans le condensat et qui sont polymérisables organiquement thermiquement ou photochimiquement, en particulier des groupes époxy.

6. Utilisation selon une des revendications précédentes, dans laquelle au moins une partie des groupes organiques présente une chaîne hydrocarbonée avec 2 à 8, de préférence avec 2 à 6, atomes de carbone, laquelle se trouve entre deux groupes choisis parmi des groupes éther, ester, amide et uréthane.

7. Utilisation selon une des revendications précédentes, dans laquelle au moins une partie des groupes organiques présente au moins deux chaînes hydrocarbonées avec 2 à 8 atomes de carbone qui sont présentes dans des chaînes ramifiées l'une avec l'autre.

8. Utilisation selon une des revendications 6 et 7, dans laquelle au moins une partie des groupes biodégradables anorganiques présente les constituants [O-(CH₂)ₘ-C(O)-O]n, où m est un nombre entier entre 2 et 8, n est égal à 0, 1, 2 ou 3 ou supérieur à 3 et, dès lors que ledit constituant est présent de manière multiple dans le groupe biodégradable organique, peut prendre des valeurs différentes dans la mesure où n est égal ou supérieur à 1 dans le seul constituant ou dans au moins dans un desdits constituants.

9. Utilisation selon une des revendications précédentes, dans laquelle les groupes organiques sont choisis dans le groupe qui est composé de polycaprolactone triol, chitosane, cellulose et des éléments constitutifs de cellulose.

10. Procédé de préparation d'un produit d'enrobage choisi parmi des produits pharmaceutiques et alimentaires enrobés, qui comprend les étapes suivantes :
(i) l'application d'une composition, qui est présente en option dans un diluant et/ou un solvant et qui contient un (hétéro)polycondensat d'acide silicique modifié avec des groupes organiques, les groupes organiques étant des polymères, sur un substrat choisi parmi des produits pharmaceutiques et alimentaires ;
(ii) le séchage et/ou le durcissement de la composition.

11. Procédé selon la revendication 10, qui comprend avant l'étape (i) ou après l'étape (ii) l'étape (iii) dans laquelle le substrat est enrobé au moins partiellement avec une couche d'oxyde métallique.

12. Procédé selon la revendication 10 ou 11, dans laquelle la composition décrite dans une des revendications 1 à 9 est utilisée.

13. Produit d'enrobage, qui peut être préparé selon le procédé des revendications 10 à 12.

14. Produit d'enrobage, qui présente un substrat choisi parmi des produits pharmaceutiques et alimentaires et un enrobage d'une composition appliqué sur le substrat et qui contient un (hétéro)polycondensat d'acide silicique modifié avec des groupes organiques, les groupes organiques étant des polymères.

15. Produit d'enrobage selon la revendication 14, dans lequel la composition indiquée dans une des revendications 1 à 9 est contenue.
